# EUROPEAN PATENT SPECIFICATION

(11) **EP 0 402 116 B1**
(45) Date of publication and mention of the grant of the patent: **25.09.1996**
(21) Application number: 90306154.7
(22) Date of filing: 06.06.1990
(51) Int. Cl.: C07K 14/00, A61K 39/12, G01N 33/569, C12N 15/40, C12N 15/86, C12Q 1/68, C12Q 1/70

(54) **Proteins, vaccines and nucleic acids**
Proteine, Vakzine und Nucleinsäuren
Protéines, vaccins et acides nucléiques

(30) Priority: 06.06.1989 EP 89305708
(43) Date of publication of application: 12.12.1990
(73) Proprietor: IMMUNO Aktiengesellschaft, A-1221 Wien (AT)
(72) Inventor: Heinz, Franz X., A-1080 Wien (AT); Mandl, Christian W., A-1160 Wien (AT); Kunz, Christian, A-1180 Wien (AT); Dorner, Friedrich, A-1230 Wien (AT); Bodemer, Walter, A-1228 Wien (AT); Antoine, Gerhard, A-2301 Gross Enzersdorf (AT)
(74) Representative: Hansen, Bernd, Dr. Dipl.-Chem.

(56) References cited:
- EP-A- 0 284 791
- WO-A-90/01946
- JOURNAL OF GENERAL VIROLOGY, vol. 70, 1989, SGM, GB; J.M. LEE et al., pp. 335-343
- NUCLEIC ACIDS RESEARCH, vol. 16, no. 15, 1988, IRL Press Ltd., Oxford (GB); V.F. YAMSHCHIKOV et al., p. 7750
- VACCINE, vol. 6, February 1988, Butterworth & Co. Ltd.; C.A. GIBSON et al., pp. 7-9
- BIOLOGICAL ABSTRACTS, vol. 87, no. 10, 1989, Biological Abstracts Inc., Philadelphia, PA (US); V.V. POGODINA et al., AN 101239
- FEBS LETTERS, vol. 200, no. 2, May 1986, Federation of European Biochemical Societies; A.G. PLETNEV et al., p. 317
- CHEMICAL ABSTRACTS, vol. 97, no. 23, 06 December 1982, Columbus, OH (US); F.X. HEINZ et al., p. 317, AN 195475s
- VIROLOGY, vol. 173, 1989, Academic Press Inc.; G.W. MANDL et al., pp. 291-301
- VIROLOGY, vol. 168, 1988, Academic Press Inc.; C.W. MANDL et al., pp. 197-205
- CHEMICAL ABSTRACTS, vol. 112, no. 21, 21 May 1990, Columbus, OH (US); A.A. KHROMYKH et al., p. 203, AN 193124f
- JOURNAL OF CHROMATOGRAPHY, vol. 502, 1990, Elsevier Science Publishers B.V., Amsterdam (NL); A.J. CROOKS et al., pp. 59-68

## Description

The present invention relates to vaccines and diagnostic agents for western subtype TBE virus, and to nucleic acid and proteins useful for such vaccines and agents.

Western subtype tick-borne encephalitis (TBE) virus is a member of the family flaviviridae, which are spherical lipid enveloped RNA viruses (Westaway et al, Intervirology **24**, 183-192, 1985). The prototype virus is yellow fever virus. By definition, all flaviviruses are serologically related as revealed by hemagglutination-inhibition assays. By cross-neutralisation, however, the family can be subgrouped into several serocomplexes (DeMadrid and Porterfield, J. Gen. Virol. **23,**, 91-96, 1974) comprising more closely related flaviviruses as opposed to serologically more distantly related viruses of different serocomplexes or ungrouped flaviviruses. TBE virus is a member of the so-called tick-borne serocomplex, which in addition also contains viruses termed Louping ill, Langat, Omsk hemorrhagic fever, Kyasanur Forest disease, and Negishi.

TBE virus strains could be further assigned to a Western (European) subtype which is primarily transmitted by Ixodes ricinus and a Far Eastern subtype with Ixodes persulcatus as its main vector (Clarke, 1964; Bull. WHO **31,** 45-56).

This subtype differentiation was confirmed by competitive RIA and peptide mapping using limited proteolysis of the corresponding structural glycoproteins (Heinz and Kunz, 1981; J. Gen. Virol. **57**, 263-274) as well as antigenic analysis using monoclonal antibodies (Heinz et al, Virology **126**, 525-537, 1983).

Mature virus particles contain only 3 structural proteins, termed E, C, and M with approximate molecular weights of 50 to 60,000, 15000, and 7000, respectively.

The genome of flaviviruses consists of single-stranded RNA of about 11000 bases with mRNA polarity having a molecular weight of 4 x 10⁶ daltons. Together with the C-protein, this RNA forms a spherical nucleocapsid which is surrounded by a lipid envelope associated with both the proteins E and M. Experiments using purified preparations of the E protein obtained after solubilization of the virus by detergents have revealed that E represents the viral hemagglutinin, ie, it causes agglutination of certain erythrocytes under appropriate conditions, which upon immunisation, induces hemagglutination-inhibiting, neutralising and protective antibodies, as well as immunity against challenge with live virulent virus (Heinz et al, Infect. Immun. **33**, 250-257, 1981).

In addition to these structural proteins, several non-structural virus-specified proteins have been found in cells infected with certain flaviviruses.

The genome organisation of flaviviruses has recently been determined by cDNA cloning and sequencing of yellow fever, West Nile and Murray Valley encephalitis virus (Rice et al, Science **229**, 726-733, 1985; Dalgarno et al, J. Mol. Biol. **187**, 309-323, 1986; Castle et al, Virology **147**, 227-236, 1985; Castle et al, Virology **149**, 10-26, 1986; Wengler et al, Virology **147**, 264-274, 1985). According to these analyses, the genome RNA of flaviviruses contains a single long open reading frame of about 11,000 nucleotides which codes for all structural and non-structural proteins.

Mandl et al. (Virology 166, 197-205, 1988) describe the sequencing of the S terminal region of the genome of Western subtype TBE virus.

The gene order established for YF virus and confirmed for several other flaviviruses is 5;C-prM(M)-E-NS1-NS2A-NS2B-NS3-NS4A-NS4B-NS5 3.C, prM(M) and E represent structural proteins found in immature (c, prM, E) and mature (C, M, E) virus particles, whereas the rest of the genome codes for non-structural proteins. The structural proteins and the non-structural proteins have been positively identified by amino-terminal sequence analysis and have thus been correlated with the corresponding genome segments.

It is presumed that translation of viral proteins initiates at the first AUG at the 5'-end of the RNA and proceeds until a stop codon is encountered at the 3'-end of the RNA. The formation of individual proteins is thought to occur by a series of specific proteolytic cleavage events involving cellular as well as virus-specified proteases.

About 60 different flaviviruses have been recognised up to now and about two-thirds of them are transmitted by the bite of infected arthropods, thus representing arthropod-borne (ARBO) viruses. Several flaviviruses are well known human pathogens and include yellow fever virus, dengue virus, Japanese encephalitis virus, or tick-borne encephalitis virus (Shope, in: "The Togaviruses", pp. 47-82, Academic Press, New York, 1980).

TBE virus is endemic in many European countries, Russia and China. The disease is well documented in some Central European countries such as Austria, Czechoslovakia and Hungary, and several hundred hospitalised cases are recorded each year. This represents a significant public health problem.

The disease can be effectively prevented by vaccination with a highly purified formalin-inactivated whole virus vaccine (Kunz et al, J Med. Virol **6**, 103-109, 1980) which induces an immune response against the structural proteins of the virus. The principal disadvantage of this vaccine is that large volumes of infectious and potentially hazardous virus suspensions have to be handled in the course of the manufacturing process. Thus extensive and expensive safety precautions are required.

Means were therefore sought for preparing a vaccine which overcame the above discussed disadvantages. EP-A-0284791 addressed the problem by providing a DNA molecule that comprises Western subtype TBE virus derived DNA, coding for at least a part of at least one structural protein, ie selected from the group comprising the proteins C, prM, M or E, of Western subtype TBE virus.

Such a DNA molecule corresponds to the single stranded RNA of Western subtype TBE virus, and proved suitable for providing genetic information for expressing a polypeptide which may be the entire protein C, prM, M or E of Western subtype TBE virus, as described above, or part of one of the above-mentioned proteins. Such a polypeptide can be used diagnostically or therapeutically for example in the preparation of a vaccine.

The sequence analysis also confirmed the differentiation from the Far Eastern subtype by revealing an amino acid sequence diversity of up to 14 (depending on the protein) as compared to the Far Eastern subtype (Yamshchikov and Pletnov, Nucleic Acid Res. **16** 7750 (1988)).

Although a vaccine based on one or more recombinant structural proteins as in EP-A-0284791 may well represent an improvement over an inactivated whole virus vaccine from the manufacturing point of view, it shares with the inactivated whole virus vaccine the disadvantage that it does not contain non-structural proteins, which may contribute to a protective immune response.

The non-structural proteins have important functions for the life cycle of flaviviruses being involved in virus maturation, proteolytic processing and RNA replication. Although one of the non-structural proteins (NS 1) has been shown for certain flaviviruses other than TBE to be capable of inducing a protective immune response (Schlessinger et al, J. Gen. Virol **68** 853-857 (1987); Zhang et al, J. Virol. **62** 3027-3031 (1988)) in experimental animals, there has been no such disclosure for non-structural proteins of TBE.

Gibson et al. (Vaccine, 6, 7-9, 1988) review the immunogenicity of the NS-1 protein of flaviviruses in general.

It is known that subneutralizing concentrations of neutralising antibodies or other antibodies to epitopes in the flavivirus E protein can mediate antibody-dependent enhancement of infectivity in Fc-receptor-bearing cells (Porterfield, Cardosa; "Concepts in Viral Pathogenesis I", Chapter 17 p.117) which has been implicated in the pathogenesis of dengue hemorrhagic fever and dengue shock syndrome (Halstead, Science **239** 476-481 (1988)). In certain cases it may therefore be advantageous to avoid the use of flavivirus E protein in vaccines or to even to use NS 1 as the only vaccine component and not to incorporate viral structural proteins.

An NS-1 protein of the Western subtype of TBE virus has now been identified by its sequence. This protein is a useful vaccine component and may conveniently be produced by recombinant DNA technology.

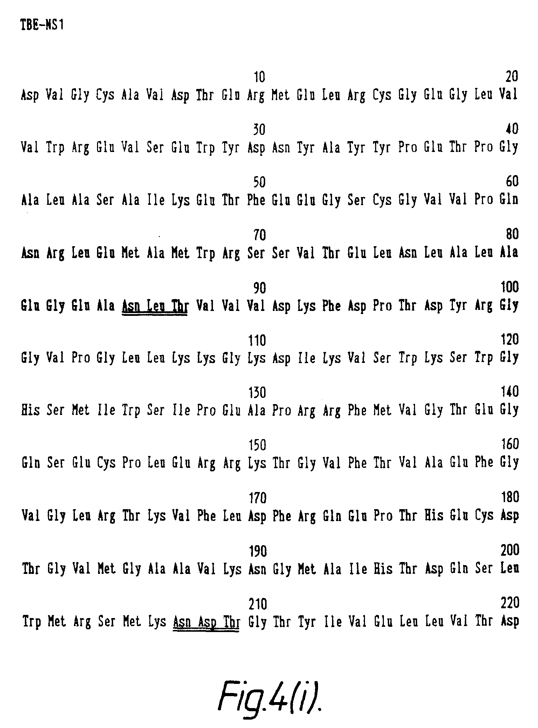

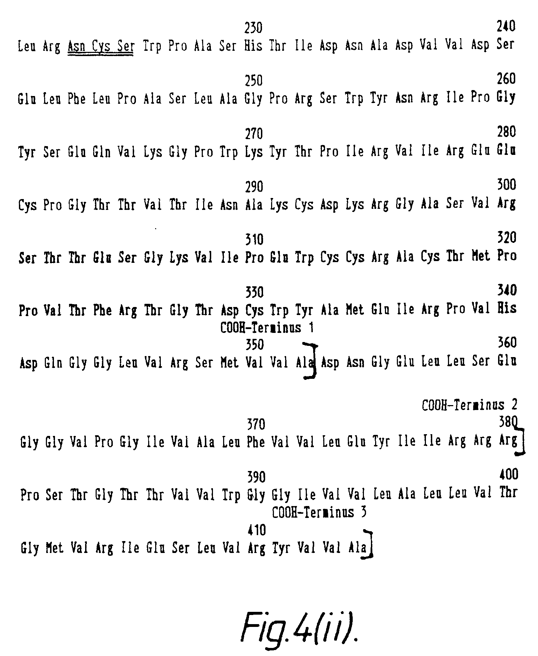

According to a first aspect of the invention, there is provided a peptide or polypeptide comprising the amino acid sequence of the NS-1 protein of Western subtype TBE virus as shown in Figure 4.

Although it is preferred to produce the peptides or polypeptides of this invention by expression of appropriate nucleotide sequences of this invention, it is also possible to isolate or, preferably, chemically to synthesise the peptides or polypeptides of the invention. The peptides or polypeptides may therefore be provided in substantially pure form and/or substantially free of other substances with which they are naturally associated.

Peptides or polypeptides according to first aspect of this invention may be useful on their own or in conjuction with one or more appropriate excipients as diagnostic agents and as ingredients in the manufacture of vaccines. Preferably the peptides or polypeptides are comprised in a composition which can be used in the medical field.

According to a second aspect of the present invention there is provided a vaccine composition comprising a peptide or polypeptide in accordance with the first aspect of the invention and one or more additional pharmaceutically acceptable components. Such components may include a pharmaceutically acceptable carrier or adjuvant for the peptide or polypeptide. Other suitable components may include a buffer.

The vaccine carrier may be any suitable carrier and may comprise one or more adjuvants if necessary. In accordance with usual practice all vaccine compositions in accordance with this invention may be sterile.

A further, preferred use of vaccines containing antigenic peptides or polypeptides is for preparing specific immunoglobulins, for example monoclonal or polyclonal antibodies, which may be prepared by methods known to those skilled in the art. Antibodies against the peptides and polypeptides may also be regarded as forming part of the invention.

As mentioned above, a composition comprising one or more of the peptides or polypeptides according to this invention may also be used as a diagnostic reagent.

The invention also relates to nucleic acid coding for peptides or polypeptides in accordance with the first aspect.

According to a third aspect of the invention, there is provided nucleic acid coding for the NS-1 protein of Western subtype TBE virus. The nucleic acid, which may and often will be recombinant nucleic acid, particularly recombinant DNA, can code for the whole, or substantially the whole of the NS-1 protein.

Not only are these DNA and RNA molecules useful for providing the peptides or polypeptides in accordance with the first aspect, they may also be used in preparing a live vaccine. Preferably the DNA sequences are combined with the DNA of Vaccinia virus which is well established as a live vaccine.

Apart from their use as live vaccines, the DNA or RNA sequences according to this invention are additionally suitable as probes for screening purposes.

At present no antiviral agents are available for the specific treatment of TBE patients. There are, however, several potential possibilities to interfere specifically with the viral life cycle including processes that involve non-structural proteins such as RNA replication, posttranslational proteolytic processing and virus assembly. Nucleic acid coding for the protein NS-1 or part of it would also be useful for this purpose. In addition to interference with functional activities of viral proteins it is also feasible to block the viral replication process by the use of anti-sense RNA.

There are a number of ways for preparing DNA molecules within the scope of this invention. One possibility of obtaining the DNA molecule is first to extract the viral RNA from Western subtype TBE virus and purify the RNA molecule, followed by transcription of this RNA template into a DNA molecule using reverse transcriptase. A further possibility is chemically to synthesise the DNA molecules according this invention, once the DNA sequence has been investigated.

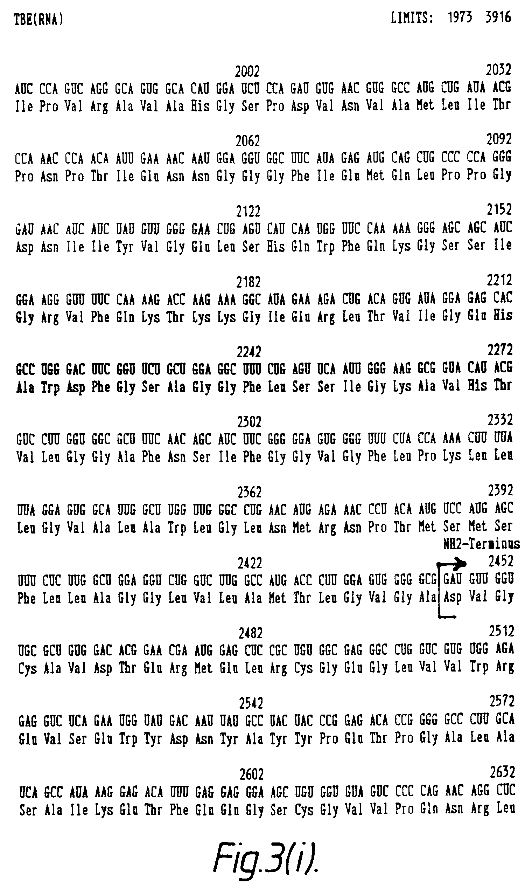

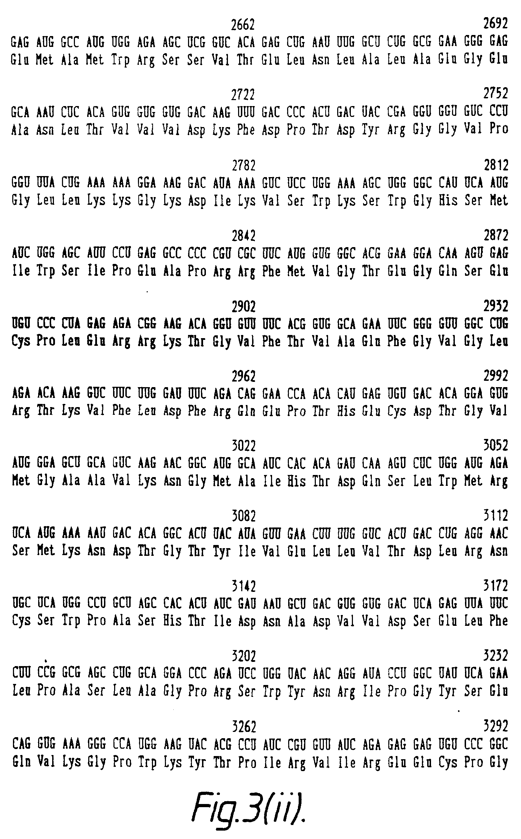

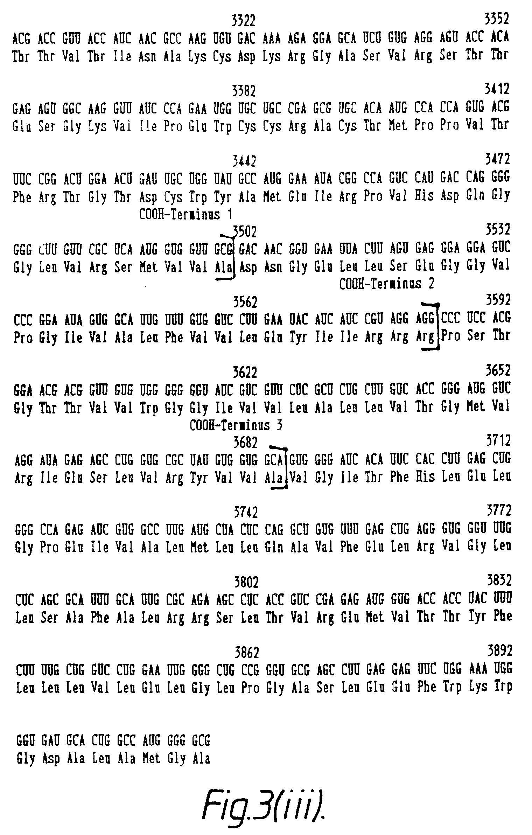

As a preferred embodiment, this invention includes DNA molecules which hybridise to DNA molecules according to the first aspect, and particularly to a DNA sequence as shown in Figure 3 and/or a DNA sequence coding for a protein sequence as shown in Figure 4, under stringent conditions, eg selecting for at least 90% nucleotide sequence homology. DNA molecules of this preferred kind may still code for peptides being able to cause antibody responses, and furthermore, those DNA molecules are suitable as DNA probes.

By one embodiment of the present invention nucleic acid including the complete sequence of wild-type Western subtype TBE virus genome coding for the non-structural protein NS-1 is provided as well as DNA molecules derived from the genomic RNA coding for the protein. DNA molecules within the scope of the invention may correspond to or be complementary with the single stranded RNA of Western subtype TBE virus, and are suitable for providing genetic information for expressing the whole NS-1 protein or one or more parts thereof which can usefully be used as a vaccine component or for diagnostic and therapeutic purposes.

The present invention includes explicitly all DNA sequences which differ from the DNA molecules corresponding or complementary to a natural NS-1 RNA sequence whether by degeneration of the genetic code and/or mutations and/or transpositions, which are in the normal range of natural variation of Western subtype TBE virus. These sequences therefore still code for proteins having the essential properties (particularly the essential antigenic properties) of the NS- 1 non-structural protein of Western subtype TBE virus.

In a preferred embodiment of this invention, the claimed DNA molecules can be combined with additional DNA sequences.

These additional sequences may allow replication and expression of the DNA molecule in a cell culture. The most important DNA sequences for this purpose are those which function as promoters, enhancers, polyadenylation signals, and splicing signals. These additional DNA sequences may be combined with the DNA molecules in accordance with the first aspect of the invention according to standard procedures known to those skilled in the art.

The advantages discussed above for DNA molecules within the scope of the invention are also true for RNA molecules in the same way. RNA molecules according to this invention may be obtained by isolation and purification of Western subtype TBE virus RNA, or by recombinant RNA/DNA techniques. Furthermore, not only are RNA molecules obtained by purification of the Western subtype TBE virus included in this invention, but also RNA molecules which have been obtained by transcribing isolated and purified virus RNA into DNA by reverse transcriptase and subsequent transcription of DNA thus obtained into RNA again, or by RNA-dependent RNA transcription.

In the present invention, not only are the DNA and the RNA molecules as described above provided, but also vectors, comprising as an insert a DNA or RNA molecule within the scope of the invention. According to a fourth aspect of the invention, there is provided a vector, which may be a cloning vector or an expression vector, comprising a nucleic acid sequence in accordance with the third aspect. The vector may for example be a plasmid, virus (for example an animal (eg vaccinia) virus or a phage) or cosmid. As is known in the art, such vectors generally comprise sequences which control replication and expression of the inserted RNA or DNA sequences; such control sequences may comprise a promoter and possibly additionally an enhancer, among other sequences.

According to a fourth aspect of the invention, there is provided a host cell containing a vector as described above. Many such cells may be provided in a cell culture.

The vectors are preferably contained in cell cultures in which the expression of the polypeptides coded for by the RNA or DNA sequences is according to this invention, the cell culture being preferably a mammalian cell culture. By using a mammalian cell culture, the most preferred conditions are provided for expression of a polypeptide which is intended to be used as a vaccine for preventing mammals from Western subtype TBE virus infections.

For a better understanding of the present invention, and to show how it may be put into effect, preferred embodiments of the present invention will now be described with reference to the accompanying drawings, in which:

FIGURE 1 shows a photograph of agarose gel electrophoresis of the undigested plasmid 85 17-6 (lane b), which contains the entire coding sequence for the NS1 protein. Lane c shows a BamHI digest of the same plasmid and the two lanes a contain 0.6Kb, 2Kb and 10Kb size markers.

FIGURE 2 shows the complete nucleotide sequence of insert 17-6, contained in plasmid BS 17-6.

FIGURE 3 shows the RNA sequence and the encoded amino acid sequence of insert 17-6. The position numbers relate to the full length genome of TBE virus.

FIGURE 4 shows the amino acid sequence of protein NS1 as derived from the sequence information of clone 17-6. Position numbers are counted from the first amino acid of protein NS1.

FIGURE 5, referred to in Example 10 below, shows a strategy for cloning NS1 into the prokaryotic expression vector pUC19S.

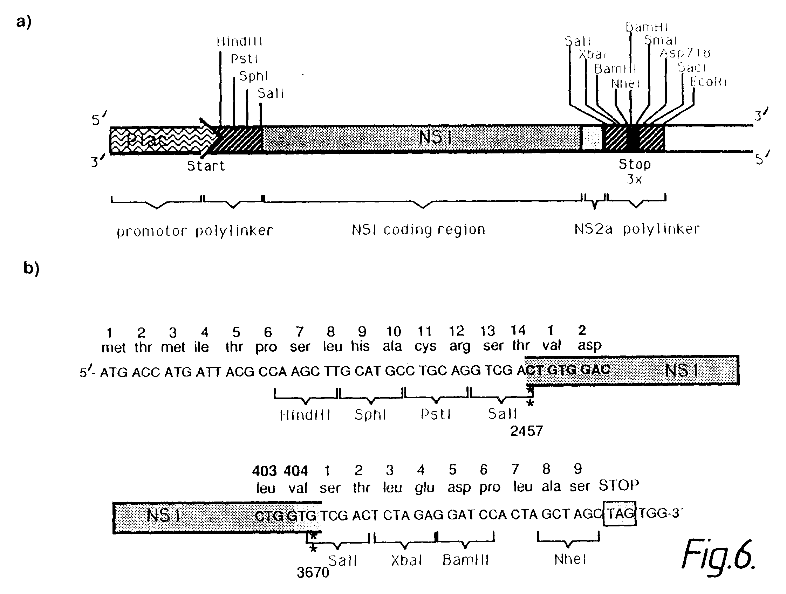

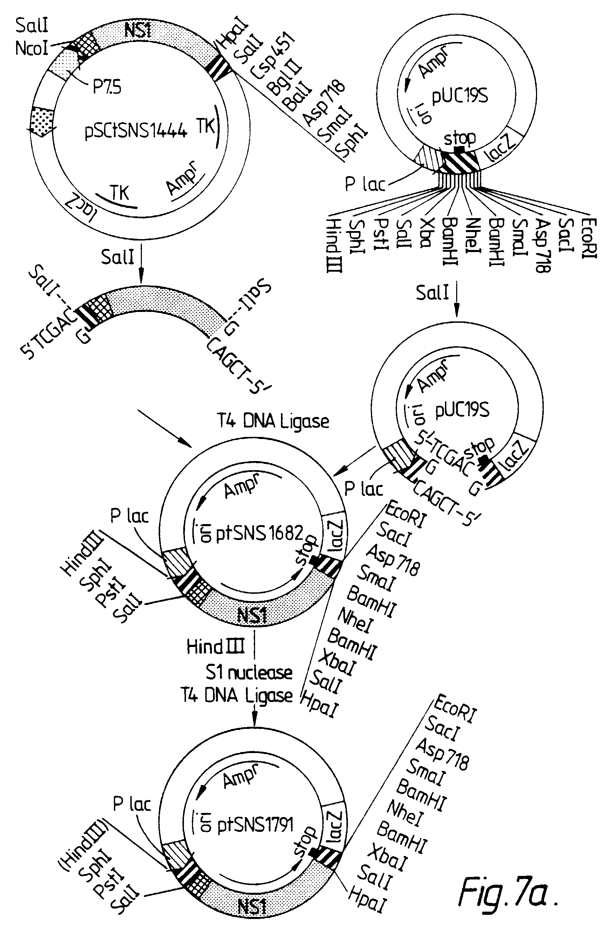

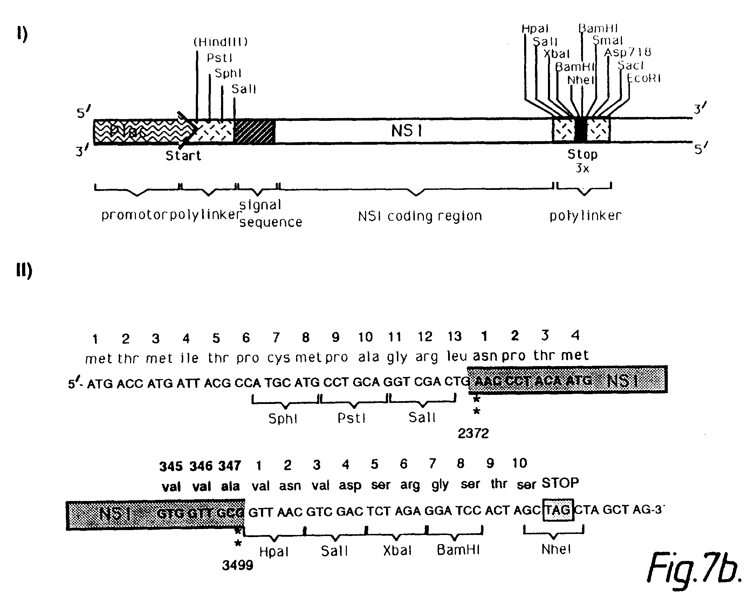

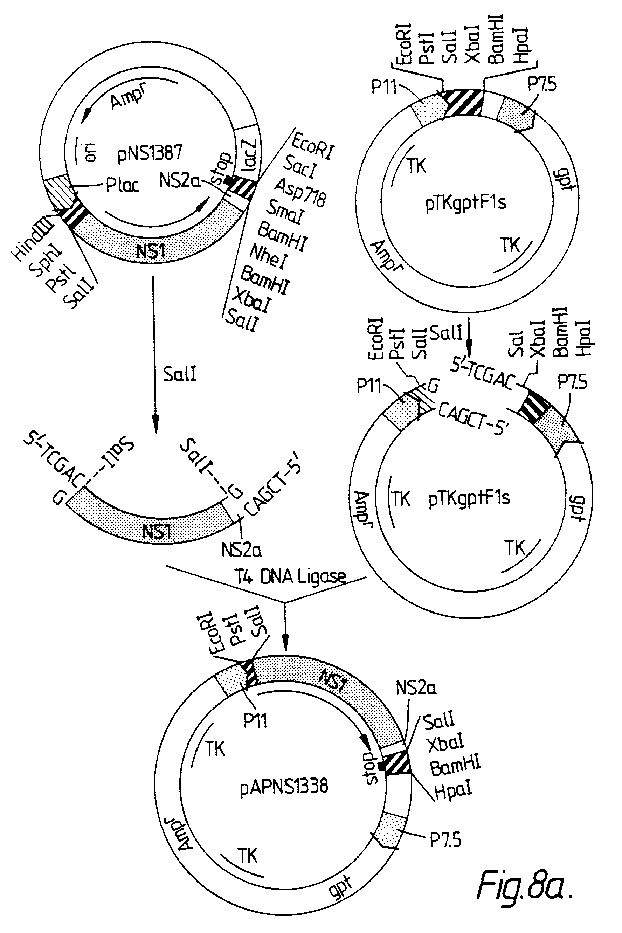

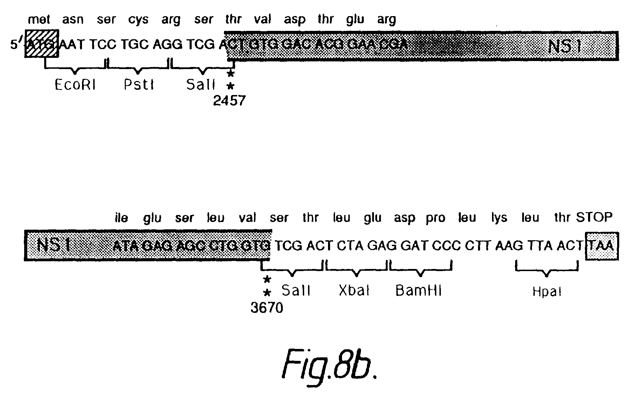

FIGURES 6A and 6B, also referred to in Example 10, show nucleotide sequences of clone pNS1387 at the 5′ and3′ termini. An NS1 insert is in frame with the bacterial lacZ gene. Expression is under the control of the lacZ operator/promoter system. In FIGURE 6A the general organisation of the construct is shown. In FIGURE 6B the DNA and protein sequences are indicated. In this construct the NS1 has 40 additional nucleotides corresponding to 14 amino acids from the lacZ gene and the pUC19S polylinker at its 5′-end. There are an additional 27 non-NS1 nucleotides (corresponding to 9 amino acids) at the 3′-end. Nucleotide positions relative to the TBE genome, taken from FIGURE 3, are indicated by asterisks.

FIGURE 7A, referred to in Example 11, shows a strategy for cloning NS1 with its authentic 5′ signal sequence into the prokaryotic expression vector pUC19S.

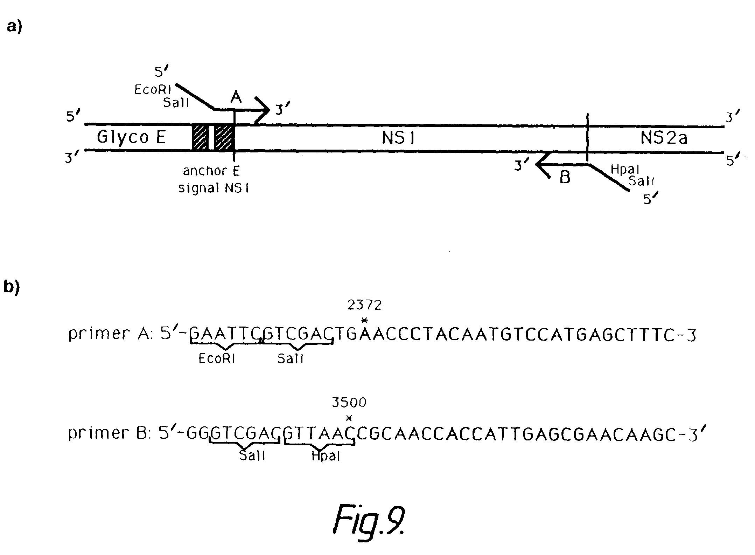

FIGURE 7B, also referred to in Example 11, shows nucleotide sequences of clone ptSNS1791 at the 5′ and 3′ termini. The NS1 insert is in frame with the bacterial lacZ gene expression under the control of the lacZ promotor system. In part I the general organization of the construct is shown. In part II DNA and protein sequences are indicated. In this construct the NS1 has 39 additional nucleotides corresponding to 13 amino acids from the lacZ gene and the pUC19S polylinker at its 5′-end. There are additional 30 non-NS1 nucleotides corresponding to 10 amino acids at the 3′-end. Nucleotides positions relative to the TBE genome, taken from FIGURE 3, are indicated by asterisks.

FIGURE 8A, referred to in Example 12, shows the cloning of the NS1 coding region into the transfer vector pTKgptF1s for recombination with vaccinia virus.

FIGURE 8B, also referred to in Example 12, shows the nucleotide sequences of clone pAPNS1338 at the 5′ and 3′ termini. The NS1 has 19 additional nucleotides corresponding to 7 amino acids in its expected translation product from the polylinker region at its 5′-end additional 30 nucleotides, corresponding to 10 amino acids, are found at the 3′-end. Asterisks indicate the nucleotide positions in the TBE Virus genome taken from FIGURE 3.

FIGURES 9A and 9B, referred to in Example 13, show the cloning of NS1 and its putative signal sequence by the polymerase chain reaction (PCR). FIGURE 8A shows a generalised strategy for the PCR. FIGURE 8B shows the sequence of chemically synthesised oligonucleotides used as primers for the DNA amplification. The asterisks indicate the nucleotide positions in the viral genome according to FIGURE 3.

FIGURE 10, also referred to in Example 13, shows an agarose gel showing the NS1 fragment synthesised by PCR. The agarose gel is stained with ethidium bromide. DNA is detected under u/v light. The arrow indicates the fragment which is about 1130 bp long.

FIGURE 11, also referred to in Example 13, shows the cloning of the NS1 coding region with its putative signal sequence after synthesis by PCR into the transfer vector pSC11-OrthDELTA0 for recombination with vaccinia virus.

FIGURE 12, also referred to in Example 13, shows the sequences of clone pSCtSNS1444 at the 3′ and 5′ termini. The authentic NS1 coding region, including its signal sequence, acquires in its expected translation product 12 additional nucleotides, corresponding to four amino acids, from the polylinker region at its 5′-end. An additional 47 nucleotides (ie 16 amino acids) are found at the 3′-end. Asterisks indicate the nucleotide positions in the TBE virus genome, taken from FIGURE 3.

FIGURE 13, also referred to in Example 13, shows an analysis of the plasmid pSCtSNS1444 by digestion with appropriate restriction endonucleases. The DNA patterns indicate the correct orientation of the inserted NS1 sequence into the pSC11-Orth vector and confirms the size of the fragments as deduced from the nucleotide sequence of clone 17-6.

FIGURE 14, referred to in Example 14, shows the cloning of NS1 with its putative signal sequence into the transfer vector pTKgptF3s, for recombination with vaccina virus.

FIGURE 15, also referred to in Example 14, shows the nucleotide sequence of the plasmid pTKtSNS1556 at the 5′ and 3′ termini. In this construct the NS1 has 24 additional nucleotides corresponding to eight amino acids in its expected translation product from the polylinker region at its 5′-end. An additional 26 nucleotides (ie 9 amino acids) are found at the 3′-end. Asterisks indicate the nucleotide positions in the TBE virus genome, taken from FIGURE 3.

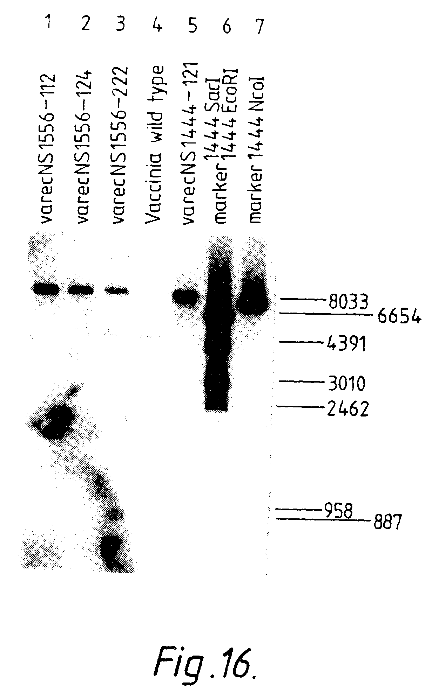

FIGURE 16, referred to in Example 15, shows a southern blot analysis of NS1 vaccinia recombinants varecNS1444 and varecNS1556. CV-1 cells infected with recombinant vaccinia viruses at a multiplicity of 5 pfu/cell. Two days after infection total cellular DNA was purified, digested with the restriction endonuclease HindIII and separated on 1% agarose gel. The DNA was transferred onto a nitrocellulose filter and hybridized with the radioactively labelled plasmid pSCtSNS1444 as a probe for the detection of the inserted NS1 fragment and adjacent viral tk sequences. A comparison of the recombinants varecNS1556-112, -124, -122 and varecNS1444-121 with wildtype Vaccinia DNA (slot 4) shows the expected upshift of the HindIII fragment towards a higher molecular weight. Only varecNS1556-222 shows a minor band corresponding to the w.t. fragment. This indicates that the plaque isloate is not homogeneous. 1556-122 etc. are different plaque isolates from the same recombination experiment after three plaque purfications. As controls and as size markers, different digests of the plasmid pSCtSNS1444 were used (slots 6 and 7).

FIGURE 17, referred to in Example 16, shows an autoradiograph of a denaturing SDS gel showing the recombinant viruses varecNS1444 and varecNS1556 expressing NS1 protein. A protein with an expected molecular weight of about 48kD can be detected which is not found in wild type or varec1342 (expressing a different protein) infected cells. The very strong protein band migrating above the 97kD marker band represents β-galactosidase (116kD) which is expressed under the control of the P11 promotor in the pSC11-Orth derived recombinants varecNS1444 and varec1342.

Nucleic acid in accordance with the present invention may be prepared and the sequence determined by the following general procedures:

First, viral RNA may be extracted from Western subtype TBE virus or Western subtype TBE virus infected cells. Using this RNA as a template, a double stranded cDNA may be synthesised, for example by reverse transcriptase. By the use of recombinant DNA techniques this cDNA may be inserted into a vector DNA such as Escherichia coli plasmid DNA to yield a recombinant plasmid. Recombinant plasmids may be used to transform appropriate host cells such as E. coli strain HB101 for amplification of the plasmids or the expression of the corresponding proteins. Individual colonies with insert-containing plasmids may be identified by the mini-plasmid preparation method according to Birnboim and Doly (Nucleic Acids Research, **7**, 1195-1204, 1979). The base sequence of the Western subtype virus specific DNA present in the recombinant plasmid can be determined by rapid DNA sequencing methods such as the dideoxy chain termination method.

A more detailed description of one particular process of producing cDNA, recombinant plasmids, cells transformed with these plasmids and of determining the nucleotide sequence is as follows: First, Western subtype TBE viral RNA may be obtained from purified virus. For this purpose, Western subtype TBE virus can be grown in primary chick embryo cells, concentrated by ultracentrifugation, and purified by two cycles of sucrose density gradient centrifugation. After solubilising the proteins with SDS in the presence of proteinase K and RNAse inhibitor, eg by incubating for 1 hour at 37°C, the RNA is extracted with, for example, phenol, and precipitated with ethanol. Using this RNA as a template, a DNA complementary to the virus RNA is then synthesised in vitro by a reverse transcriptase, eg from avian myeloblastosis virus, for instance by the method of Gubler and Hofmann (Gene **25**, 263-269, 1983). Using primers, such as hexanucleotide primers obtained from calf thymus DNA, the viral RNA is incubated under appropriate conditions, eg as described in the manual "cDNA synthesis system" from Amersham, England, with a reverse transcriptase together with deoxyadenosine triphosphate (dATP), deoxythymidine triphosphate (dTTP), deoxyguanosine triphosphate (dGTP), and deoxycytidin triphosphate (dCTP) as substrates. The thus obtained cDNA.RNA hybrid is treated with RNAse H under defined conditions which generates nicks in the RNA of the hybrid molecule. E. coli DNA polymerase I can then be used efficiently to replace the RNA strand utilising the nicked RNA as primers. The double-stranded RNA thus obtained is deproteinised by phenol-chloroform extraction, precipitated with ethanol and remaining RNA fragments are removed by treatment with RNAse (eg in TE buffer, 37°C, 30min).

Cloning of the dsDNA is performed, eg by the use of synthetic linkers. For this purpose, the dsDNA is treated with the Klenow fragment of E. coli DNA polymerase I under appropriate conditions (Maniatis et al, Molecular Cloning, CSH 1982) to ensure a maximum amount of clonable blunt ends, followed by phenol extraction for deproteinisation. dsDNA is incubated under appropriate conditions with BamHI linkers in the presence of DNA ligase. The reaction mixture is loaded and run on a 1% agarose gel and different size classes of cDNA are cut out of the gel and purified, eg by electroelution. On the other hand, a plasmid DNA to be used as vector DNA, eg E. coli plasmid BLUESCRIPT SK-, is cut with the restriction endonuclease BamHI and dephosphorylated by the use of bacterial alkaline phosphatase. After digestion of the cDNA with BamHI it is mixed with the BamHI -cut vector DNA and incubated at appropriate conditions to allow for hybridisation of the complementary overhanging sequences at the ends of both DNA molecules and ligated by the use of T4-DNA ligase. The recombinant DNA molecule is used to transform a competent E. coli strain (eg E. coli XL 1-blue). Recombinants containing virus specific inserts are identified by colour selection. Plasmids are isolated by the mini-plasmid preparation method (Birnboim and Doly, Nucleic Acids Research **7**, 1195-1204, 1979) and insert sizes are analysed by restriction enzyme analyses using BamHI and separation of the fragments obtained on 1% agarose gels.

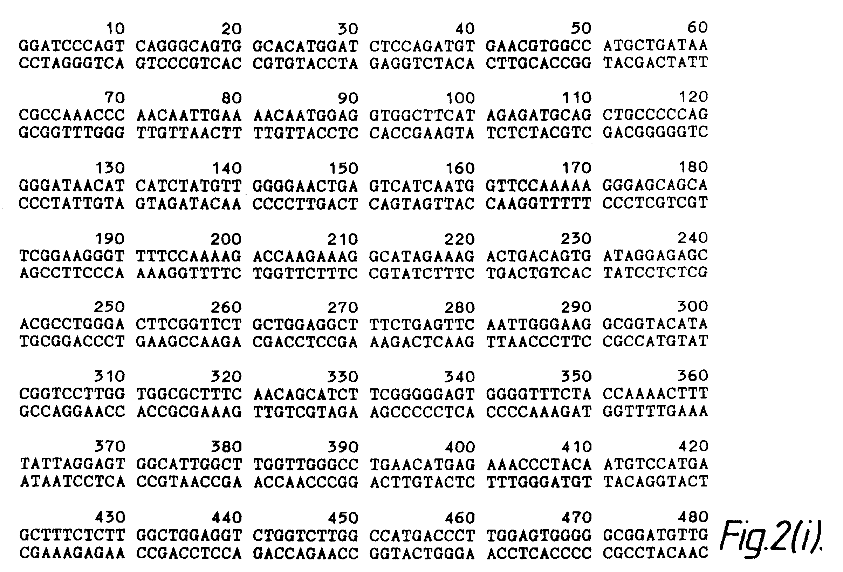

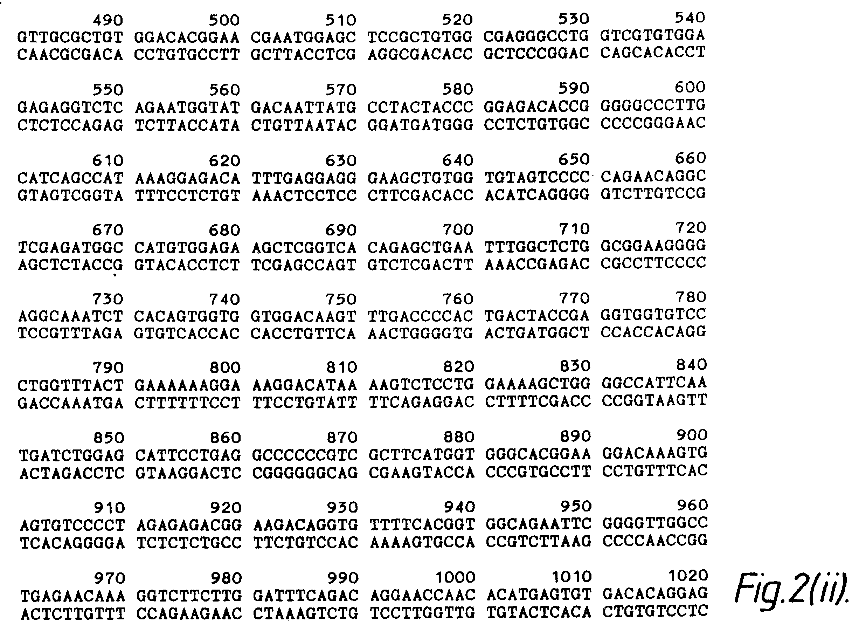

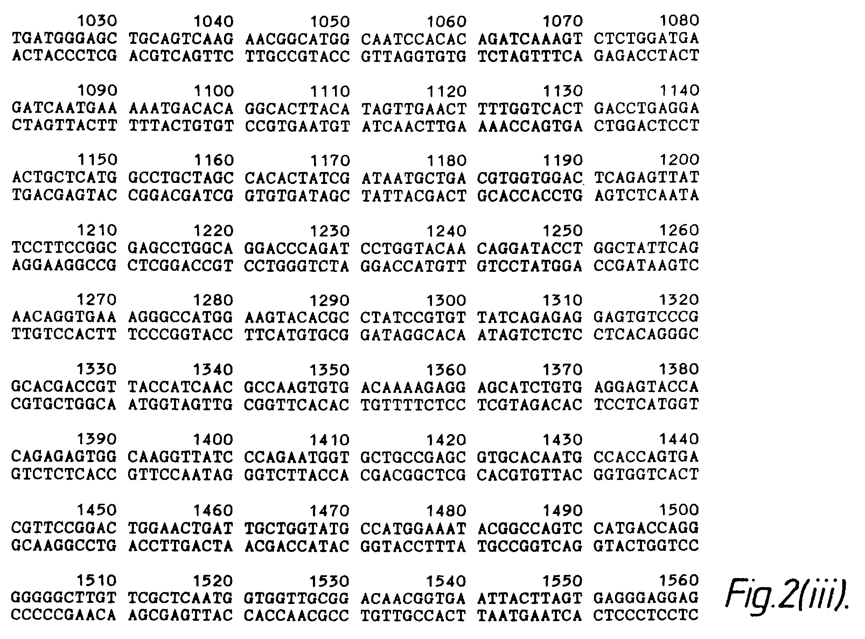

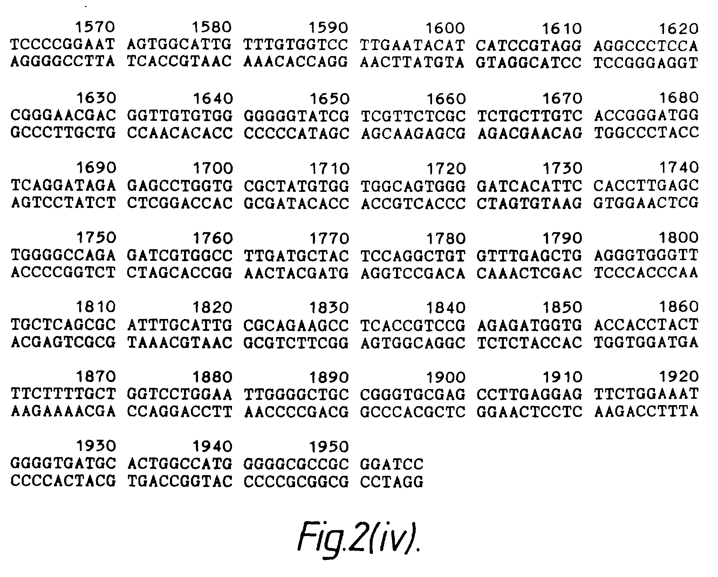

The base sequence of these inserts is determined by the dideoxy chain termination method according to Sanger et al (PNAS USA, **74**, 5463-5467, 1977). The sequence obtained is analysed for homologies with already known sequences of other flaviviruses (Rice et al, Science **229**, 726-733, 1985; Dalgarno et al, J. Mol. Biol, **187**, 309-323, 1986; Castle et al, Virology **147** 227-236, 1985; Castle et al, Virology **149**, 10-26, 1986; Wengler et al, Virology **147**, 264-274, 1985; Yamshchikov and Pletnev Nucleic Acid Res. **16** 7750, 1988) by the aid of computer programs in order to determine the location of the sequence under investigation in the total sequence of the genome RNA. Figure 2 shows the complete nucleotide sequence and Figure 3 the corresponding amino acid sequence of the NS1 region of the western subtype TBE virus genome.

The present invention therefore for the first time provides the nucleotide sequence of the gene coding for the NS1 protein of the Western subtype TBE virus and, as a consequence, also provides the amino acid sequence of this protein.

The base sequence in Figure 2 is a preferred example for a DNA which codes for polypeptides having the characteristics of the Western subtype TBE virus NS1 protein. Due to the degeneration of the genetic code, the same amino acid sequence may also be coded for by base triplets other than those shown in the Figure.

Within the scope of protection of this invention there are also sequences included which are altered by mutation, transposition and degradation, which nevertheless code for an amino acid sequence still having the essential antigenic characteristics of the NS1 protein.

In addition, the invention does not only relate to exactly the same amino acid sequence as that shown in Figure 3, which represents only an exemplary sequence of the NS1 sequence of a natural Western subtype TBE virus isolate. It is a well-known fact that the genomes of RNA viruses are subject to higher mutation frequencies than those found with DNA viruses or cellular genes due to the high error rate of RNA polymerases and the lack of proof-reading mechanisms (Holland et al, Science **215**, 1577-1585, 1982; Reanney, Ann. Rev. Microbiol. **36**, 47-73, 1982). Depending on the characteristics of the virus, these mutations may give rise to the rapid development of new virus types due to antigenic drift, as is the case with influenza virus (Both et al in: "The Origin of Pandemic Influenza Viruses", W.G. Laver (ed.), Elsevier, 1983). On the other hand, RNA viruses can be antigenically more stable under natural ecological conditions, presumably due to functional constraints which do not allow for extensive structural changes in antigenically active proteins. Nevertheless, a certain degree of variation has to be taken into account and can be demonstrated by sequence comparison of different natural isolates.

This can be done for example by sequencing the RNA of different isolates using the dideoxy chain termination method with synthetic oligonucleotides as primers which have been prepared according to the sequence of the prototype isolate shown in Figure 2.

All sequences which, compared to the prototype sequences, show slight variations such as those found in other natural isolates of Western subtype TBE virus are included in this invention. Such variations may also be obtained by in vitro modification of the nucleic acid. The invention therefore includes all sequences which hybridise under stringent conditions, eg selecting for at least 90% nucleotide sequence homology with the sequences or parts of the sequences disclosed and which preferably code for proteins or peptides having the essential antigenic determinant characteristics of the Western subtype TBE virus structural proteins.

Insertion of the nucleotide sequence coding for a protein such as NS-1 into appropriate vector(s) and host cells for expression at high level is a powerful technology for the study of the structure and biological function(s) of the protein. The expression system to be used is always dependent on certain molecular characteristics e.g. post-translational modification of the protein molecule of interest.

Expression can be performed in prokaryotic or eukaryotic cell systems. For glycoproteins such as NS-1 expression would better be performed in a eukaryotic host cell capable of proper glycosylation.

A preferred expression system consists of vaccinia virus and a primate cell growing continuously in culture. This ensures, or helps to ensure, that correct post translational modifications and high level synthesis can be achieved.

Furthermore there is ample evidence that gene regulatory sequences of vaccinia virus can direct the synthesis of almost any foreign protein either using a "late phase" promoter or using a "constitutive" promoter active during the early and late phase of viral replication (Moss and Flexner, Ann. Rev. Immunol. **4** 305-324 (1987). However, it has been reported that the stability of foreign proteins expressed in vaccinia virus-infected cells is strongly influenced by the promoter regulating the expression of the gene of interest (Coupar et al., Eur.J.Immunol. **16** 1479 (1986); Townsend et al., J.Exp-Med. **168** 1211-1224 (1988)).

The construction of appropriate expression plasmids containing NS-1 specific inserts requires extensive DNA manipulation. It has to be taken into account that NS-1 is part of a polyprotein which is post-translationally processed into individual proteins by cellular and viral protcases (Mandl et al., Virology **173** 291-301 (1989)). In the course of a natural virus infection the synthesis of NS-1 involves an internal signal sequence at the NH₂-terminus. The signal directs the nascent polypeptide chan into the lumen of the endoplasmic reticulum, which is necessary for proper post-translational modifications such as glycosylation. In order to obtain a correctly processed expression product, this or a heterologous signal sequence has to be included in the recombinant molecule.

Furthermore, since NS-1 is part of a polyprotein it does not contain start and stop codons for translation. These have to be provided by specific recombinant DNA manipluations.

Hydrophobic (5′) signal and (3′) anchor sequences are included in the sequence of clone 17-6 (Figure 3). The lack of appropriate endonuclease restriction sites does not allow straightforward cloning of NS-1 with its "natural" signal sequence into insertion vectors. Therefore, a stretch of 1200 bp comprising the NS-1 signal sequence and the sequence of the mature NS-1 may be synthesised, for example, in a polymerase chain reaction (PCR). This technique also allows the introduction of optional restriction sites on both sides of the amplified DNA, as well as start and signal sequences.

A specifically designed primer-pair for the amplification results in an NS-1 sequence product flanked by recognition sites for the restriction endonuclease EcoRI. Using this methodology it is possible to exchange the authentic NS-1 signal sequence by any signal sequence naturally occuring or by a synthetic consensus signal sequence according to the proposals by v.Heinje (NAR **14** 4683-4690 (1986)).

All plasmids, specifically developed for the expression of NS-1 molecules, can be transposed into a parental vaccinia virus (strain WR or attenuated derivates of the same) by homologous recombination in permissive host cell e.g. primate CV-1 cells. The experimental procedure to generate recombinant vaccinia viruses is common knowledge, is well known to those skilled in the art and does not need to be described (Mackett et al. JRL Press; "DNA cloning II" Ed. DM Glover 191-211, 1985).

The present invention also relates to the synthesis of NS-1 molecules in a bacterial host. Glycoproteins expressed in bacterial cells are usually not glycosylated in the same manner as in eukaryotic cells. Nevertheless synthesis of NS-1, even in a non-glycosylated form, may be useful for different purposes. As an example, derivatives of the envelope glycoprotein (gp) 160 of HIV-1 expressed in bacterial cells have significantly contributed to HIV-diagnosis and experimental vaccination.

Analogous to the construction of insertion plasmids for recombination with vaccinia virus an NS-1 sequence including its authentic signal sequence is synthesised with the PCR and cloned into, for example, pUC-19. A substitution of the TBE signal sequence by a prokaryotic signal sequence in order to obtain efficient secretion of NS-1 molecules from the bacterial cells is possible. The latter may prevent the aggregation of NS-1 and formation of inclusion bodies that have to be dissolved with detergents or other solubilising agents.

Besides the synthesis of complete and mature NS-1 this experimental protocol can also be applied to truncated forms of the NS-1 molecule. Truncated molecules can be used to induce an immune response directed to specific sites of the protein. Such forms can also be valuable tools for performing "site directed serology". Immunologically relevant epitopes within the NS-1 may become incorporated into carrier molecules as adjuvants (e.g. HB-core particles) or into an immunstimulating complex (ISCOM) (Morein et al. Immunology Today **8** (11) 333-338 (1987)).

Any of the NS-1 molecules synthesised in prokaryotic and eukaryotic expression systems described in this application represent molecules suitable as candidate NS-1 vaccine. either alone or perhaps by combining it with the recombinant glycoprotein-E of TBE-virus (EP-A-0284791) or the already available vaccine consisting of inactivated TBE-virus.

Further, the analysis of the immune response against this non-structural protein or parts thereof may be critical for understanding the pathogenicity of the TBE-virus and other members of the flavi-virus family.

Foreign DNA or RNA sequences can also be engineered into the genomes of live viruses thus generating recombinant viruses which can be used as a live vaccine (for review, see Mackett and Smith, J. Gen. Virol. **67**, 2067-2082, 1986).

Also combinations of different genes, eg derived from different viruses, can be simultaneously expressed by recombinant DNA technologies and used for vaccination (Perkus et al, Science, **229**, 981-984, 1985). The invention therefore includes any combinations of sequences out of the sequences disclosed with other sequences, such as genes coding for other proteins or sequences contributing to the expression of the proteins, such as promoters, enhancers, polyadenylation or splicing signals.

It is well known to those skilled in the art that not necessarily the whole naturally occurring proteins have to be used for immunisation or diagnostic purposes (Lerner, Nature **299**, 592-596, 1982; Arnon, TIBS **11**, 521-524, 1986).

Proteins or parts of proteins to be used as vaccines or diagnostic reagents can not only be prepared by the recombinant DNA technologies described above, but the sequence information disclosed in the present invention can also be used for the chemical synthesis of oligopeptides. There is extensive literature available on this subject: peptides synthesised according to DNA sequences coding for many different proteins have been prepared and used for a variety of purposes such as molecular biological and immunological studies (Lerner et al, Cell, **23**, 309-310, 1981; Lerner, Nature, **299**, 592-596, 1982) or vaccination (Shinnick et al, Ann. Rev. Microbiol. **37**, 425-446, 1983; DiMarchi et al, Science, **232**, 639-641, 1986). The preparation and use of peptides or combinations of peptides corresponding to sequences disclosed in the present invention therefore represent the state of the art.

It is further known to those skilled in the art to use the nucleic acids and sequences provided by the present invention for the preparation of hybridisation probes eg for the use of determining virus RNA in ticks or blood-fluid (Meinkoth and Wahl, Anal. Biochem. **138**, 267-284, 1984; Kulski and Norval, Arch. Virol. **83**, 3-15, 1985). These can be prepared either by recombinant DNA technologies or by the chemical synthesis of oligonucleotides according to the sequence disclosed.

The invention will now be illustrated by the following examples. Where not specifically stated, procedures used follow those conventionally used in the art. Such procedures may be found in a number of standard works, including Sambrook, Fritsch and Maniatis "Molecular Cloning: A Laboratory Manual" (second edition), Cold Spring Harbor Laboratory Press, 1989.

### EXAMPLES

### Example 1 Propagation and Purification of TBE Virus

A 10% suspension of TBE virus-infected suckling mouse brain was used for the infection of primary chick embryo cell monolayers maintained in minimum essential medium (MEM) buffered with 15mM HEPES and 15mMEPPS at pH 7.6. After 40h incubation at 37°C the supernatant was clarified at 10000g for 30min at 4°C and the virus was pelleted by ultracentrifugation at 50000g for 3h at 4°C. The virus was then resuspended in an appropriate volume of TAN buffer (0.05M triethanolamine, 0.1M NaCl, pH 8.0) and subjected to rate-zonal centrifugation in a 5-20% (w/w) sucrose density gradient at 170000xg for 110min at 4°C. The virus peak was located by scanning the gradient at 254nm and subjected to equilibrium density gradient centrifugation in a 20 to 50% (w/w) sucrose gradient for 18 h/4°C at 150000xg. The virus peak was dialysed against TAN pH 8.0 to remove excess sucrose.

### Example 2 Preparation of Viral RNA

100µg of purified virus were diluted into 400µl of proteinase K reaction buffer (10mM Tris pH 7.8, 5mM EDTA, 0.5% w/v SDS). Proteinase K was added to a final concentration of 200µg/ml and the mixture was incubated for 1hr at 37°C. Subsequently the solution was deproteinized by extracting it twice with equal volumes (400µl) of phenol and once with chloroform: isoamyl alcohol (24:1). then 26µl of a 3M Na-Acetate solution were added and the RNA was precipitated with 2.5 volumes of ethanol. Before further use, an aliquot of the RNA was denatured with glyoxal and run on a 1% agarose gel in order to check the yield and the quality of the preparation.

### Example 3 Synthesis of Double Stranded cDNA

5µg of ethanol precipitated RNA were resuspended in 40µl of first strand synthesis buffer (Amersham, UK) that contained 5µg of random oligonucleotide primers, heated to 70°C for one minute and then allowed to cool slowly to room temperature. All four deoxynucleotide triphosphates were added to final concentrations of 1mM. Furthermore, 5 units of human placental ribonuclease inhibitor and 10µCi of α-³²P dCTP were added to the mixture. First strand synthesis was started by the addition of 100 units of reverse transcriptase (Amersham) and allowed to proceed for 2 hours at 42°C. Then the reaction mixture was placed on ice and the reagents for the second strand synthesis were added in this order: 93.5µl of second strand synthesis buffer (Amersham, UK), 4 units of ribonuclease H, 23 units of E. coli DNA polymerase and water to a final volume of 250µl. Second strand synthesis was carried out by subsequent incubations at 12°C and 22°C (2 hours each) and stopped by heating the solution for 20 minutes to 70°C. The double stranded cDNA was digested with 10 units of T4 DNA polymerase for 30 minutes at 37°C in order to create blunt ends. Finally the cDNA was purified by phenol and chloroform extractions and precipitated with 2 volumes of ethanol.

### Example 4 Linker Ligation

Synthetic 5′-phosphorylated BamHI linkers (New England Biolabs) were added onto the cDNA in an overnight reaction at 12°C. The reaction mixture contained in a final volume of 20µl of ligation buffer (50mM Tris pH 7.5, 5mM MgCl₂, 5mM DTT, 1mM ATP) approximately 1µg of cDNA, 1µg of linker DNA and 1µl of T4 DNA ligase (New England Biolabs).

### Example 5 Size Fractionation of cDNA

The cDNA was fractionated on a 1% agarose gel. Different size fractions were cut out of the gel and the DNA was extracted from the agarose in an analytical electroeluter (IBI) according to the manufacturer's directions. In spite of the small amount of DNA handled, which could hardly be detected by ethidium bromide staining, the extraction procedure could easily be monitored due to the radioactive label incorporated into the cDNA. The size fractionation step provided a means of selectively cloning large fragments of cDNA and also served to completely separate the cDNA from unligated linker molecules.

### Example 6 Cloning into a Bacterial Phagemid

The cDNA was digested with 10U of BamHI restriction endonuclease for 1hr at 37°C. After phenol and chloroform extractions the DNA was precipitated with 2 volumes of ethanol and resuspended in a small volume of water. 20 - 30ng of the cDNA were mixed with 50ng of the phagemid BLUESCRIPT SK- (Stratagene) that had been linearized by BamHI digestion. (The word BLUESCRIPT is a trade mark.) These two components were litigated by T4 DNA ligase in 10µl of ligation buffer (see above) in an overnight reaction at 16°C. The next morning, the ligation mixture was heated to 65°C for 5min, diluted hundredfold with water and used to transform E. coli XL1 Blue cells. Competent bacteria were purchased from Stratagene and transformed with 3µl of the diluted ligation mixture exactly following the protocol provided by the manufacturer. Bacteria were plated onto LB agar plates containing ampicillin, tetracyclin, IPTG and X-Gal.

### Example 7 Identification of Insert-Containing Phagemids

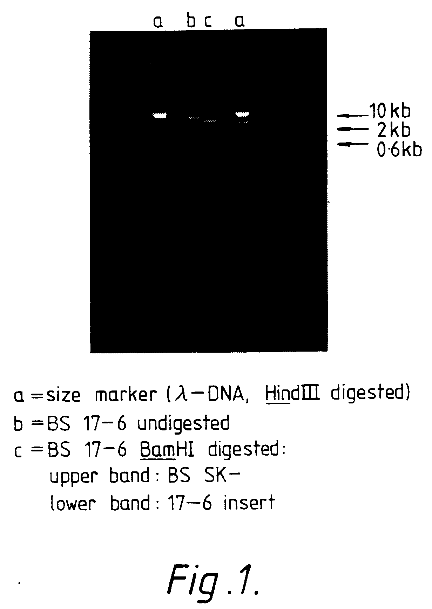

In the BLUESCRIPT vector system insert-containing phagemids yield white bacterial colonies, whereas self-ligated phagemid induces a blue colour reaction. Thus white colonies were picked and used to inoculate 2ml of LB-Medium containing ampicillin and grown overnight at 37°C in a shaker at 220 rotations per minute. The next day a quick plasmid preparation was performed by a standard boiling method (Birnboim and Doly, Nucleic Acids Research **7**, 1195-1204, 1979). Plasmids were digested with the restriction enzyme BamHI and analysed on 1% agarose gels. One clone identified in this manner was phagemid BS 17-6, that carries an approximately 2000 bp long virus specific insert. Figure 1 shows a 1% agarose gel of a quick plasmid preparation of BS 17-6 both in the supercoiled circular and the BamHI digested forms.

### Example 8 Sequence Analysis of the Insert of Clone BS 17-6

Single stranded DNA of the clone BS 17-6 was prepared by addition of the VCS helper phage (Stratagene) at a M.O.I. of 20:1 to exponentially growing cultures of 85 17-6 containing E. coliXL1 Blue bacteria. After agitating vigorously the culture at 37°C for 16 hrs ssDNA was purified from the supernatant according to standard protocolls. This single-stranded DNA was sequenced in the whole region of the insert by the dideoxy method of Sanger et al (PNAS USA **74**, 5463-5467; 1977) using the SEQUENASE enzyme (United States Biochemicals) and the reagents supplied by the enzyme-manufacturer. (The word SEQUENASE is a trade mark.) A vector specific and a set of virus-sequence specific oligonucleotides were used as primers for the sequencing reactions. The complete sequence of the insert 17-6 is shown in Figure 2.

### Example 9 Derivation of the Amino Acid Sequence of Protein NS1

Translation of the nucleotide sequence of 17-6 in all possible reading frames revealed only one long open reading frame. The amino acid sequence derived in this frame was aligned to sequences of other flaviviruses (Rice et al, Science **229**; 726-733 (1985); Coia et al, J. Gen. Virol. **69**, 1-21 (1988); Chambers et al, Virology **169**, 100-109 (1989)). For these analyses the Beckman MICROGENIE Software (Version 4.0) was employed on an IBM personal computer. The word MICROGENIE is a trade mark. The analyses indicated that clone 17-6 contained the entire coding sequence for protein NS1 and parts of the coding sequences of the proteins E and NS2A which flank the NS1 gene within the flaviviral genome.

Figure 3 depicts the RNA sequence and the encoded amino acid sequence derived from clone 17-6. The amino terminus of NS1 is supposed to be released by a cellular signalase, the carboxy-terminus by a signalase-like enzyme. A larger form of the NS1 protein that contains also the amino-terminal part of the protein NS2A was observed by Mason et al (Virology **158**, 361-372 (1987)). The carboxy-terminus of this form of the NS1 protein may be liberated by a virus-specified protease or the cellular signalase (Rice et al, Science **229**, 726-733 (1985)). The amino-terminus and three possible carboxy-termini are indicated in Figure 3. The carboxy-terminus liberated by a signalase-like enzyme (Chambers et al., Virology **169** 100-109 (1988)) is depicted as "COOH-Terminus 1˝; the carboxy-terminus possibly formed by a probably virus-specified protease is indicated as "COOH-Terminus 2˝; and the potential carboxy terminus liberated by the cellular signalase is shown as "COOH-Terminus 3". The position numbers used in Figure 3 relate to the full length genome of TBE virus.

Figure 4 shows the amino acid sequence of protein NS1 as derived from the sequence information of clone 17-6. The possible COOH-Termini are indicated as in Figure 3. Position numbers are counted from the first amino acid of protein NS1. Figure 4 also depicts the locations of the three potential N-glycosylation sites present in the TBE virus protein NS1.

### Example 10 Cloning of the NS1 coding region into a prokaryotic expression vector

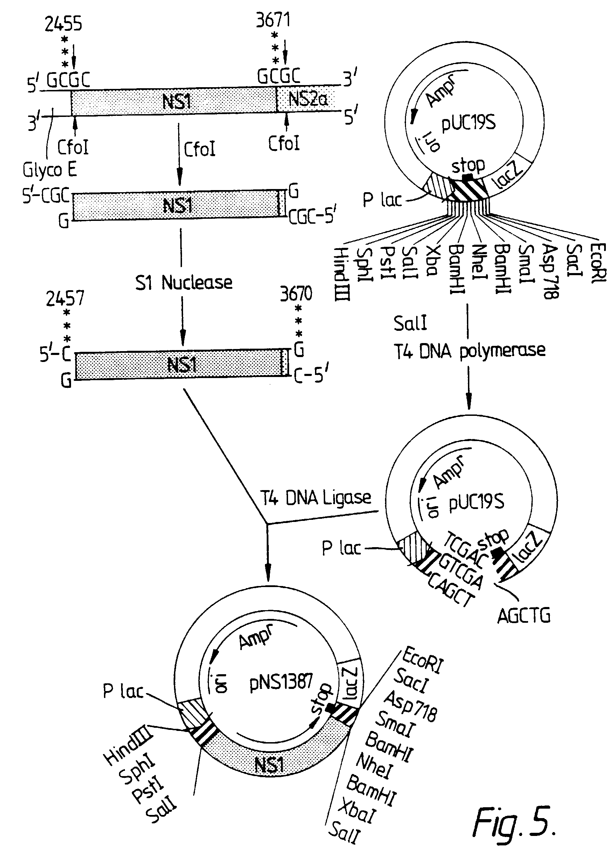

The nucleotide sequence coding for NS1 is subcloned from clone 17-6 into pUC19. The vector pUC19S is a derivative of pUC19S containing a stop linker with a TAG stop codon in all three reading frames cloned into the BamHI site. The NS1 sequence containing a small part of NS2a at its 3′-end is excised with restriction endonuclease CfoI and blunt ends are generated with S1-Nuclease. The cloning vector pUC19S is linearized in its polylinker with SalI and termini are filled-in with T4 DNA Polymerase (Figure 5). Ligation of vector and insert regenerates the SalI sites and fuses NS1 in frame to the lacZ gene (Figures 6A and 6B). At the 3'terminus the NS1 nucleotide sequence is flanked by the prokaryotic transcriptional and translational stop signals contained in the vector. The plasmid is designated pNS1387.

### Example 11 Cloning of the NS1 Coding Region including its 5′ proximal hydrophobic signal sequence using the Polymerase Chain Rection

In order to construct a induceable prokaryotic expression plasmid containing the NS1 coding region including its authentic signal sequence the SalI fragment from pSCtSNS1444 (Example 13) is transferred to pUC19S resulting in clone ptSNS1682. To place the NS1 gene into the same reading frame as lacZ, clone ptSNS1682 is digested with HindIII, blunt ends are generated with S1 Nuclease and religated thus generating clone ptSNS1791 with a deletion of 4 nucleotides (Figures 7a and 7b).

### Example 12 Construction of a plasmid for recombination into Vaccinia Virus: the NS-1 protein under the control of the VV-p11 (L) promotor)

The NS-1 coding region (eg. in Example 10) is subcloned into the SalI site of plasmid pTKgptF1s (Falkner, G.F. and B. Moss, (J. Virol **62**,1849-1854 (1988)). The NS1 sequence is positioned in frame with the ATG translational start codon of the vector. Translational stop signals are also provided by the vector. The polylinker region of the vector adds an additional 7 aminoacids at the 5'end and 10 aminoacids at the 3'end. The cloning strategy and the flanking sequences of the protein are shown in Figure 8a and 8b. The plasmid is designated pAPNS1338.

### Example 13 Construction of a plasmid for recombination into Vaccinia Virus the NS-1 protein with its natural signal sequence under the control of the VV-D7.5 (E/L) promotor

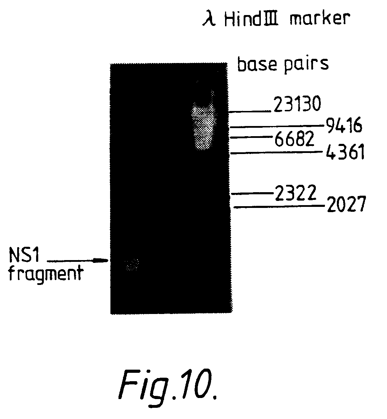

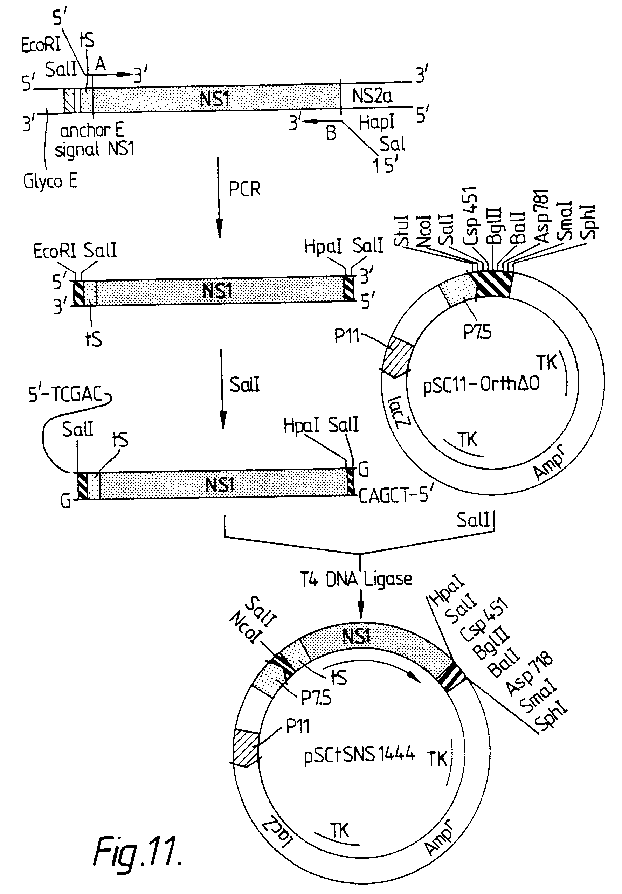

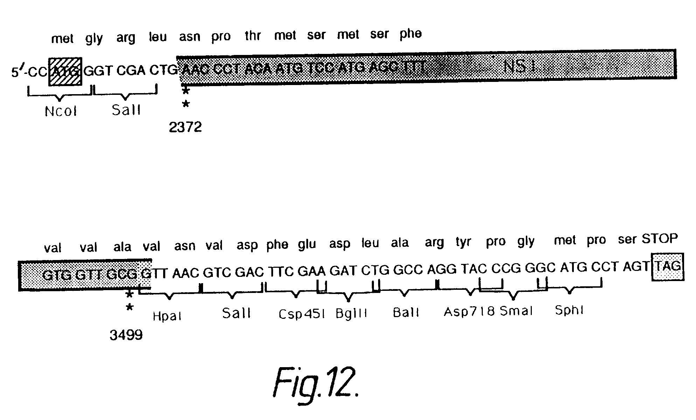

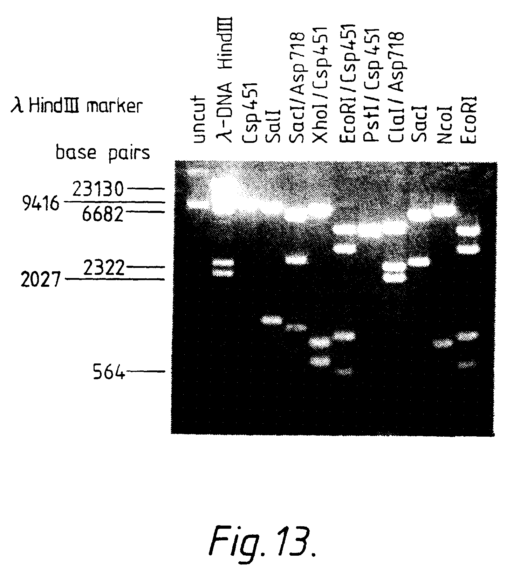

A nucleotide sequence comprising the NS1 coding region and its signal sequence is synthesized by the Polymerase Chain Reaction using clone 17-6 as a template. Two oligonucleotides with the restriction endonuclease cleavage sites EcoRI and SalI at the 5'end and SalI and HpaI at the 3'end are chemically synthesized and used as primers in PCR (Figs 9A and 9B). The amplified NS1 fragment is purified from an agarose gel (Fig. 10) cleaved with SalI and cloned into the SalI site of the vector pSC11-Orth after minor modifications. The plasmid pSC11-Orth has been deposited at the D.S.M., Braunschweig, West Germany, as DSM 5734 on 15th January 1990. The cloning strategy and sequences of the 5′ and 3′ termini of the new construct are shown in Figures 11 and 12. An agarose gel of a restriction digest of the clone confirming the correct orientation of the insert is shown in Figure 13. The plasmid is designated pSCtSNS1444.

### Example 14 Construction of a plasmid for recombination into vaccinia virus: the NS-1 protein with its original signal sequence under the control of the VV-p11 (L) promotor

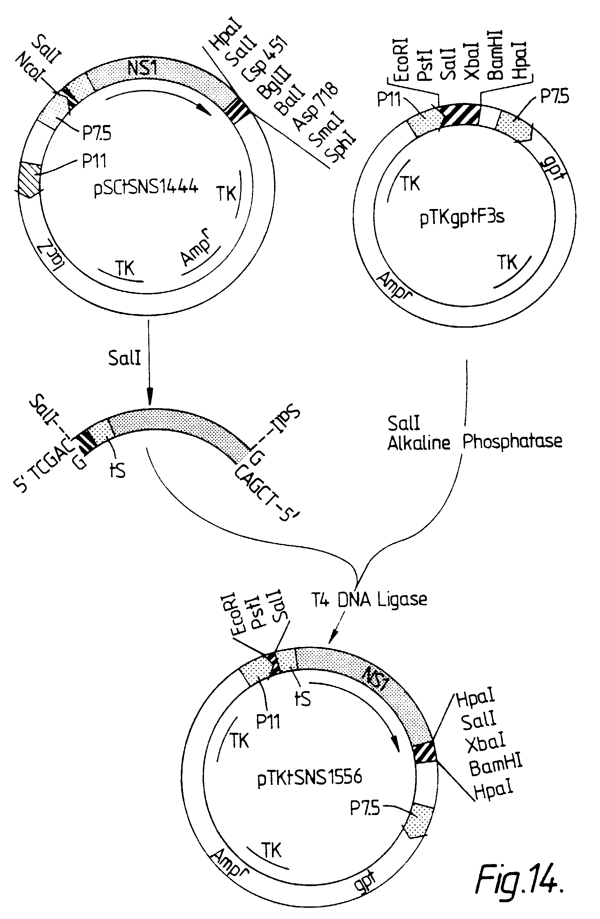

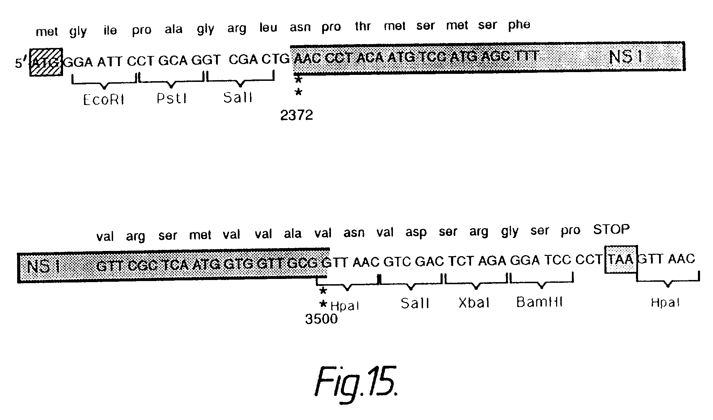

Plasmid pTKtSNS1556 results from subcloning of NS1 from clone pSCtSNS1444 into the vector pTKgptF3s (Falkner and Moss (J. Virol **62,**1849-1854 (1988)). The NS1 sequence is exised with SalI and inserted into the SalI site of pTKgptF3s. The cloning strategy and nucleotide sequences of pTKtSNS1556 are shown in Figures 14 and 15.

All plasmids are grown under ampicillin selection of 100 µg/ml in E. colihosts like DK1, BH101 or JM strains (JM105, JM83).

### Example 15 Generation of Vaccinia Recombinants for Expression to NS1 protein

Recombinant viruses are generated according to standard procedures (Mackett, M., Smith, G. and Moss, B. In: DNA Cloning: A Practical Approach, pp 191-121. Edited by D.M. Glover; Oxford: IRL Press). The plasmid DNA of pSCtSNS14444 and of pTKtSNS1556 are transfected into CV-1 cells infectedwith vaccinia virus wild type. The pSC11-Orth-derived recombinant varecNs1444 is selected for with BuDR on 143tk-cells. Th pTKgptFs derived recombinant varecNS1556 is isolated by gpt selection on CV-1 cells. All recombinants were plaque purified three times. The correct organization of the recombinants is analysed by Southern blot analysis (Fig. 16).

### Example 16 Expression of NS1 in CV-1 cells infected with Vaccinia virus recombinants

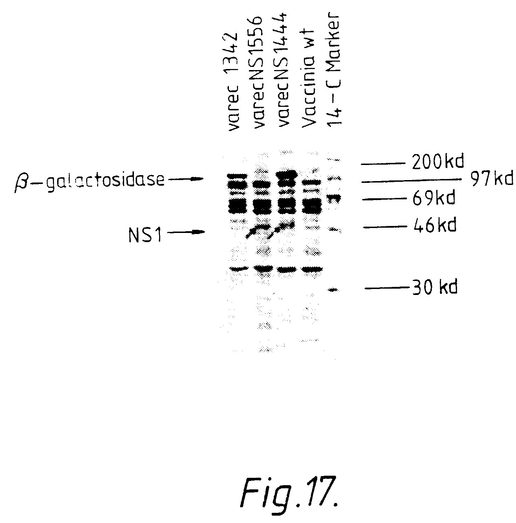

CV-1 cells are infected with the vaccinia virus recombinants varecNS1444 and varecNS1556. Two days after infection the cells are labelled with 35-S methionine for 4 hours. Proteins are separated on a 12% SDS denaturing polyacrylamide gel and identified by autoradiography. A protein band corresponding to an expected molecular weight of about 48 kD can be detected. It is missing in cells with wild type virus or non-NS1 recombinants like varec1342 (Fig. 17).

## Claims

1. A recombinant protein comprising the amino acid sequence cf the NS-1 protein of a Western Subtype TBE virus as shown in Figure 4.

2. A protein according to claim 1 for use in medicine.

3. A vaccine composition comprising a protein according to claim 1 and one or more additional pharmaceutically acceptable components.

4. A diagnostic reagent comprising a protein according to claim 1.

5. Nucleic acid coding for a protein according to claim 1.

6. Nucleic acid according to claim 5 which is DNA and which comprises additional DNA sequences wherein the additional sequences allow replication and expression of the DNA molecule in a cell culture and preferably comprise sequences which function as a promoter, an enhancer, a polyadenylation signal and/or a splicing signal.

7. Nucleic acid according to claim 6 wherein the promotor comprises the VV-p7.5 or VV-p11 promotor.

8. A vector, such as a plasmid, virus other than the naturally occurring Western Subtype TBE virus or phage, comprising nucleic acid according to any one of claims 5 to 7.

9. A vector according to claim 8, which is derived from vaccinia virus.

10. A vector according to claim 8, which is derived from a bacterial plasmid.

11. A host cell containing a vector according to any one of claims 8 to 10.

12. A culture of host cells according to claim 11.

13. A culture according to claim 12 which is a mammalian cell culture.

14. A culture according to claim 13 which is a CV-1 cell culture.

15. A method for the production of NS-1 protein of Western-subtype TBE virus, characterized in that said NS-1 protein is isolated from a culture according to any one of claims 12 to 14.

## Patentansprüche

1. Rekombinantes Protein, umfassend die Aminosäure-Squenz des NS-1-Proteins eines TBE-Virus vom westlichen Subtyp, wie in Figur 4 gezeigt.

2. Protein nach Anspruch 1 zur Verwendung in der Medizin.

3. Impfstoffzusammensetzung, umfassend ein Protein nach Anspruch 1 und ein oder mehrere pharmazeutisch verträgliche Komponenten.

4. Diagnostikum, umfassend ein Protein nach Anspruch 1.

5. Nukleinsäure, kodierend für ein Protein nach Anspruch 1.

6. Nukleinsäure nach Anspruch 5, die eine DNA ist, und die zusätzliche DNA-Sequenzen umfaßt, worin die zusätzlichen Sequenzen die Replikation und Expression des DNA-Moleküls in einer Zellkultur erlauben, und vorzugsweise Sequenzen enthält, die als Promotor, Enhancer, Polyadenylierungssignal und/oder Splicingsignal fungieren.

7. Nukleinsäure nach Anspruch 6, worin der Promotor den VV-p7,5- oder VV-p11-Promotor umfaßt.

8. Vektor, wie ein Plasmid, ein anderes Virus als das natürliche TBE-Virus vom westlichen Subtyp oder ein Phage, umfassend die Nukleinsäure nach einem der Ansprüche 5 bis 7.

9. Vektor nach Anspruch 8, der von Vaccinia-Virus abgeleitet ist.

10. Vektor nach Anspruch 8, der von einem bakteriellen Plasmid abgeleitet ist.

11. Wirtszelle, enthaltend einen Vektor nach einem der Ansprüche 8 bis 10.

12. Kultur von Wirtszellen nach Anspruch 11.

13. Kultur nach Anspruch 12, welche eine Säugerzellkultur ist.

14. Kultur nach Anspruch 13, die eine CV-1-Zellkultur ist.

15. Verfahren zur Herstellung von NS-1-Protein des TBE-Virus vom westlichen Subtyp, dadurch **gekennzeichnet,** daß das NS-1-Protein aus einer Kultur nach einem der Ansprüche 12 bis 14 isoliert wird.

## Revendications

1. Protéine recombinante comprenant la séquence d'amino-acides de la protéine NS-1 d'un virus de la TBE du Sous-Type Ouest telle que représentée dans la Figure 4.

2. Protéine selon la revendication 1, pour l'utilisation en médecine.

3. Composition de vaccin comprenant une protéine selon la revendication 1 et un ou plusieurs constituants supplémentaires pharmaceutiquement acceptables.

4. Réactif de diagnostic comprenant une protéine selon la revendication 1.

5. Acide nucléique codant pour une protéine selon la revendication 1.

6. Acide nucléique selon la revendication 5, qui est l'ADN et qui comprend des séquences d'ADN supplémentaires, dans lequel les séquences supplémentaires permettent la réplication et l'expression de la molécule d'ADN dans une culture de cellules et comprennent de préférence des séquences qui font office de promoteur, d'amplificateur, de signal de polyadénylation et/ou de signal d'épissage.

7. Acide nucléique selon la revendication 6, dans lequel le promoteur comprend le promoteur VV-p7,5 ou VV-p11.

8. Vecteur tel qu'un plasmide, un virus autre que le virus de la TBE du Sous-Type Ouest naturel ou un phage, comprenant un acide nucléique selon l'une quelconque des revendications 5 à 7.

9. Vecteur selon la revendication 8, qui est obtenu à partir du virus de la vaccine.

10. Vecteur selon la revendication 8, qui est obtenu à partir d'un plasmide bactérien.

11. Cellule hôte contenant un vecteur selon l'une quelconque des revendications 8 à 10.

12. Culture de cellules hôtes selon la revendication 11.

13. Culture selon la revendication 12, qui est une culture de cellules de mammifère.

14. Culture selon la revendication 13, qui est une culture de cellules CV-1.

15. Procédé de préparation de la protéine NS-1 du virus de la TBE du Sous-Type Ouest, caractérisé en ce que cette protéine NS-1 est isolée à partir d'une culture selon l'une quelconque des revendications 12 à 14.
